# EUROPEAN PATENT APPLICATION

(11) **EP 1 716 866 A1**
(43) Date of publication of application: **02.11.2006**
(21) Application number: 04802609.0
(22) Date of filing: 28.12.2004
(51) Int. Cl.: A61K 39/39

(54) **PROTEOLIPOSOMES AND DERIVATIVES THEREOF AS CYTOTOXIC RESPONSE-INDUCING ADJUVANTS AND RESULTING FORMULATIONS**

(30) Priority: 30.12.2003 WO PCT/CU03/00312
(71) Applicant: Instituto Finlay, Centro de Investigacion-Produccion de vacunas y sueros, La Coronela, La Lisa, Ciudad Habana 11600 (CU); Universidad de Cambridge, Facultad para las Ciencias Veterinarias, Cambridge Cambridgeshire CB3 0ES (GB)
(72) Inventor: PEREZ MARTiN, Oliver Germán, Ciudad de La Habana 11300 (CU); LASTRE GONZALEZ, Miriam de San Juan Bosco, Ciudad de La Habana 11300 (CU); RODRiGUEZ RAMIREZ, Tamara, Regla Ciudad de la Habana 11200 (CU); BRACHO GRANADO, Gustavo Rafael, Ciudad de la Habana 11500 (CU); MASTROENI, Pietro, Cambridge, Cambridgeshire (GB); DEL CAMPO ALONSO, Judith Monica, Ciudad de la Habana 10400 (CU)
(74) Representative: Gil-Vega, Victor
(86) International application number: PCT/CU2004/000017
(87) International publication number: WO 2005/063287

(57) **Abstract**

The invention relates to the field of vaccine compositions for the effective treatment or prevention of fungal, viral, protozoan or bacterial (preferable intracellular) infections and cancer. The technical objective of the invention is to obtain adjuvants for use in prophylactic or therapeutic vaccines using bacterial proteoliposomes and derivatives thereof, which, when applied, together with antigens that are inserted in, conjugated to or mixed with same, induce a cytotoxic T-lymphocyte response to the aforementioned antigen. In this way the invention can be used to obtain multiple formulations of proteoliposome or the derivatives thereof with heterologous antigens, which, when applied by parenteral or mucosal (preferably nasal) routes, induce cytotoxic responses. Said novel use of proteoliposomes and the derivatives thereof and the resulting antigen formulations can be used to produce novel vaccines in the pharmaceutical industry.

## Description

### DESCRIPTIVE MEMORY

The invention relates to the vaccine field and its use in medicine. It is particularly related to the use of adjuvants and the resulting vaccine formulations.

The intended technical objective is to increase immune responses against fungal, viral, protozoan, bacterial or cancer antigens, particularly to augment the induction of cytotoxic T-cell responses that are essential against these antigens. This approach will lead to the development of prophylactic or therapeutic vaccine formulations.

To achieve the aforementioned technical objective, the use of proteoliposomes and the derivatives thereof is proposed, as adjuvants in vaccine formulations containing fungal, viral, bacterial, protozoan or carcinogenic antigens, inserted in the proteoliposome structure, conjugated to or mixed with the same. These formulations would extend the preferential Th1 immune response induced by the proteoliposome and its derivatives to the included antigens (Pérez O et al. Infect Immun. 2001, 69(7):4502-4508), inducing an immune response mediated by T cytotoxic lymphocytes (CTL) against the said antigens being administered by mucosal or parenteral routes or the combination thereof.

### STATE OF THE ART

Infections by intracelullar fungi, virus, bacteria or protozoa frequently cause pathologies all over the world. Cancers are also a terrible scourge of all humanity. These infections and cancer of human cells imply the production of a high quantity of antigens in the cellular citosol, many of which are driven to the cellular surface associated with the molecules of the Major Histocompability Complex (MHC) class I (Heemels, M.-T. and H. Ploegh, 1995. Generation, translocation, and presentation of MHC class I-restricted peptides. Annu. Rev. Biochem. 64,463-491; Rock, K.L. 1996. A new foreign policy: MHC class I molecules monitor the outside world. Immunol. Today 17, 131-137; Jondal, M., R. Schirmbeck, and J. Reimann. 1996. MHC class I restricted CTL responses to exogenous antigens. Immunity 5:295-302; and Reimann, J. and S. H. E. Kaufmann. 1997. Alternative antigen processing pathways in anti-infective immunity. Curr. Opin. Immunol. 9:462-469)

The immune response has been phenotipically divided in cellular (Th1) and humoral (Th2). The Th1/Th2 patterns are distinguished firstly by the cytokines secreted by the T CD4⁺ lymphocytes: mainly IFNγ and IL12 by Th1 and IL4 and IL5 by Th2 (Mossman, T. R., Cherwinski, H., Bond, M. W., Giedlin, M. A, and R. L. Coffman. 1986. Two types of murine helper T cells clone. I. Definition according to profiles of lymphokine activities and secreted proteins. J. Immunol. 136:2348-2357). Besides, it is also possible to evaluate the type of induced response by determining the class and subclass profiles of serum immunoglobulins characteristics of each type of response. Thus, in mice the Th1 pattern induces mainly antibodies of the IgG2a subclass (dependent on IFNγ), whilst Th2 induces IgE and IgG1 subclass (dependent on IL4). In humans, the Th1 induces the IgG1 and IgG3 subclass and the Th2 induces the IgE class.

Other cytokines produced by non-lymphoid cells have also been associated with these patterns, as occurs with the IL2 and IL18 that are associated with a Th1 pattern. On the other hand, IFNγ and IL10 act as inhibitors of the Th2 and Th1 response, respectively.

These classifications have also now been extended also to the T CD8⁺ lymphocytes which have been classified as Tc1 and Tc2 mainly based in cytokines production similarities. T CD8⁺ lymphocytes are the main mediators and inductors of CTL activity. These act then, not only as effector cells but also as regulatory cells producing cytokines which develop one type of response more than the other. Therefore, the determination of lymphocyte subsets T CD4⁺ or T CD8⁺ present in lymphoprolipherations of periferic mononuclear cells of immunized subjects or secondary lymphoid organs of immunized animals, re-stimulated by an antigen *in vitro,* is important evidence that these two populations are being stimulated.

It is necessary to eliminate the cells infected with intracellular pathogens or the tumorous cells. The cytotoxic activity of T CD8+ lymphocytes (CTL) is one of the most efficient immune mechanisms in this respect. These lymphocytes recognize antigens presented in MHC-I context and are capable of killing the infected cells through the liberation of perforins, the production of granzymes and Fas-FasL interactions (O'Hagan D., Mackicham M., and Singh M. Recent advance in adyuvants for infection disease Biomolecular Engineering 2001). CTL activity is the most commonly accepted immune response for determining the effectiveness of vaccines against intracellular infections caused by virus, bacteria or protozoa, as well as against tumours.

Satisfactory CTL activity can be determined by any of the direct or indirect methods known preferably by combining them. Indirect ones determine the capacity of the Proteoliposome or its derivatives containing antigens inserted in their structure, conjugated or coadministered to them being phagocytosed, degraded and presented in the cellular surface of cell lines associated with cellular line MHC-I molecules. These are then co-cultured with cytotoxic cell lines specific against the inserted antigen and the cellular death is determined by radioactive or non radioactive methods. Another indirect sign is the stimulation by the proteoliposome or its derivatives containing antigens inserted in their structure, conjugated to or coadministered, of T CD8+ lymphocytes in periferic mononuclear cells of immunized humans or secondary lymphoid organs of immunized animals, re-stimulated *in vitro* with the antigen of interest. The detection of production of IFNγ and IL2 by T CD8+ lymphocytes present in mononuclear cells re-stimulated *in vitro,* with the antigen of interest, could be another indirect way, and can be detected by Flow Cytometry or ELISPOT assay. In this case mainly the production of IFNy by the T CD8+ lymphocytes is determined. IL2 production by T CD8+ hybridoma cells after recognition of the specific antigen in the context of MHC-I molecules in the cellular surface of an antigen presenting cell previously incubated with the proteoliposome or its derivatives containing the specific antigen inserted in their structure, conjugated or coadministered to them, is another indirect way of proving CTL activity.

In the direct CTL response measuring methods, mammalians are immunized with the proteoliposome or its derivatives and antigens of interest, inserted in their structure, conjugated or coadministered to them and subsequently after immunisation, the secondary lymphoid organs are extracted to determine the presence of CTL activity against the specific antigen associated with MHC-I molecules. The oldest method to measure antigen specific CTL activity is the radioactive cromium release cytotoxic assay. It has been designed for fresh cells (measuring effector CTL activity) as well as for CTL cell lines (measuring reactive CTL of memory). In both, the target cells express the specific antigen of interest. It can be achieved through different methods (infection with a recombinant virus, peptide loading or genetic transfection).More recently, previously determined specific CTL epitops have been used.

Another *ex vivo* method is the tetrameric-binding assay (TBA). Tetrameric complexes of MHC-I molecules charged with specific epitops directly bind the TCRs of antigen specific T CD8+ cells independently of its functional abilities. HLA-A2 tetramers bound to the CTL eiptops of interest re generally used. The TBA is more exact and precise for reflecting the number of *in vitro* freed T CD8+ antigen specific cells, but its ability to quantify functional antigen-specific T CD8+ cells is limited.

CTL have been considered as the main element of the cellular response for a long time. Today it is known that although these are induced during the Th1 response, not all Th1 responses strictly imply induction of CTL lymphocytes. Thus, it is necessary to find adjuvants capable of inducing a Th1 pattern of response that includes CTL activity.

Adjuvants are substances that potentiate the specific immune response against an antigen causing a faster induction of it and augmenting its duration (Vogel FR. Dev. Biol. Stand. 1998,92:241-248). Its use in vaccine formulations means that the amount of necessary antigen may be reduced, the response towards a desired pattern may be directed and the number of necessary doses may be lowered.

Among the available adjuvant systems that induce Th1 response are:
1. MF59 (emulsion of oil in microfluidized water of squalene oil). Podda, A, del Guidice G. Expert Rev Vaccines 2003 Apr.; 2 (2): 197-203.
2. Monophosphoril lipid A (MPL), lipopolisacharide derivative from *Salmonella minnesota,* which contains 6 oily acids, particularly MPL 3-D-O acetilated (3D-MPL) or other non toxic derivatives from lipopolisacharide (LPS) and combinations of MPL, preferably 3D-MPL or non toxic derivatives from LPS with aluminium salt.
3. Immunostimulatory fractions of *Quillaja* saponaria: Quil A incorporated into immunostimulatory complexes (ISCOM®) saponin complexes, cholesterol and phospholipids (Polakos NK, Drane D, Cox J, Ng P, Selby MJ, Chien D, O'Hagan DT, Houghtan M and Paliard X. J Immunol 2001,166:3589-98).
4. Particles of polilactil co-glicolidos (Putney SD and Burke PA, Nat BIOTECHNOL. 1998, 16:153-157).
5. MPL and saponin, particularly triterpenic glycoside Saponin removed from the bark of the Molina saponaria Quillaja tree QS21 and 3D-MPL as is revealed in WO 94/00153.
6. QS21 and cholesterol as is revealed in WO 98/33739.
7. QS21, 33D-MPL and tocoferol emulsified in water and oil as is revealed in WO 95/17210.
8. CpG Oligonucleotids, sequences of cytokine-guanine dinucleotide with non methylated cytokines, only characteristic of bacterial deoxyrhybonucleic acid (AND), as is revealed in WO 96/02555.

Adjuvants inductors of CTL are currently limited. Among them are the MF59, the oglionucleotides ICpG, the QS21, the MPL®, the ISCOM® and the chocleates derivated from lipids (O'Hagan D., Mackicham M., and Singh M. Recent advance in adyuvants for infection disease. Biomolecular Engineering 2001 and Edelman Robert. The development and use of vaccine adyuvant. Molecular Biothecnology 2002. 21:2, 129-148.).

Proteoliposomes have been described for the preparation of prophylactic vaccines against infectious diseases by Ruegg CL et al. (Preparation of proteosome-based vaccines. J Immunological Methods 1990;135:101-109); Lowell et al. (Proteosome-Lipopeptide Vaccines: Enhancement of Immunity for Malaria CS Peptides. Science 1988;240:800-2) and also it was revealed in US No. 5,597,572. In the last one, the main core is a proteoliposome derivated from the external membrane of *Neisseria meningitidis* serogroup B, whose particulate structure, the content of native LPS incorporated and not free, the presence of polysaccharide from *Neisseria meningitidis* serogroup C, its lipidic composition and its adsorption in aluminium, are related to its high immunogenicity and probed protection in humans.

The vaccine based on this proteoliposome, VA-MENGOC-BC™, has been applied in more than 50 million doses demonstrating that it is safe, non reactogenic and effective to protect against *N. meningitidis* serogroups B and C. It may also be applied during breastfeeding in a safe and effective way, turning the polysacharide C from T independent to T dependent antigen (Perez O. et al. Th1 response induced by the B component of VA-MENGOC-BC™ overcomes the thymus independence of polysaccharide C and primes for memory in toddler. Biotecnologìa Aplicada 2002; 19(1-2):54). This vaccine induces a preferential Th1 pattern of response characterized by the induction in humans and animals of lymphoproliferation; anti-Proteoliposome IgG antibodies; IgG1 subclasses in human and IgG2a in mice; IFNγ, IL-2 and IL-12 in the ribonucleic acid message level (mRNA) and proteins, non induction of IgE antipropteoliposome nor increases in the IgE total and non production of IL-4 and IL-5 neither as protein nor at the level of mRNA (Pérez O et al. Infect Immun. 2001, 69(72001):4502-4508). The proteoliposome and its derivatives as VSSP and cochlear structures have been also used as adjuvants as revealed in (US 6,149,921 of 2000), and in (Método de obtención de estructuras cocleares. Composiciones vacunales y adyuvantes basados en estructuras cocleares y sus intermediarios. OCPI. 2002-0292 of 27/11/02) respectively

The invention aims at employing natural or genetically modified bacterial proteoliposomes especially those derived from *N. meningitidis,* as well as cochlear structures derived thereof, as new adjuvants inducers of CTL response. These new adjuvants are of particular importance against fungal, viral, bacterial and protozoan infections (mainly intracellular) as well as cancer.

"Proteoliposome" means that they are obtained from bacterial strains by using any known method such as: its isolation without detergent, a process including detergent (such as desoxicholate) or extraction from "bleb" vesicles present in culture supernatants and those particularly revealed in US 5,597,572. Proteoliposomes contain different patogen associated molecular patterns (PAMP), molecular structures preserved between pathogens, capable of strongly stimulating the immune system.

"Cochlear structures" means adjuvants derived from Proteoliposomes that therefore contain PAMP as is revealed in the patent (Método de obtención de estructuras cocleares. Composiciones vacunales y adyuvantes basados en estructuras cocleares y sus intermediarios. OCPI. 2002-0292 of 27/11/02).

The mechanisms of protection against *Neisseria* (extracellular Gram negative bacteria) are related to the induction of antibodies that mainly mediate bactericidal and opsonophagocitic functions. Thus, it may appear unlikely that the proteoliposomes derivated from these bacteria and the natural infection produced by these bacteria, may induce CTL responses. Laboratory studies have surprisingly demonstrated, however, that humans immunized with VA-MENGOC-BC®, anti- *N. meningitidis* B and C vaccine, show activation and proliferation of both T CD4⁺ and T CD8⁺ specific lymphocytes against the proteoliposome, on evaluation in re-stimulated *in vitro* periferic blood mononuclear cells.

Also, it has been surprising to find that Balb/c mice immunized with VA-MENGOC-BC® with Proteoliposomes or cochlear structures induce specific T CD8⁺ lymphocytes responses besides the known T CD4⁺ responses.

Also it has been unexpectedly found, that the natural infection with the microorganism *N. meningitidis* serogroup B induces T CD4⁺ as well as T CD8⁺ response against Proteoliposome. These responses were detected in lymphocytes from individuals cured from meningoccocal desease caused by *N. meningitidis B.*

It was also unexpected that also mice inoculated with *N. meningitidis* B induced T CD8⁺ lymphocyte responses.

It has been found that both in cells of those immunised withVA-MENGOC-BC® and those coming from individuals cured from meningoccocal desease, the T CD4⁺, and surprisingly T CD8⁺ lymphocytes secrete IFNγ and IL-2 but not IL-4 nor IL-5.on evaluation by flow cytometry.. These results include T CD8⁺ lymphocytes amongst the cells which are important in the production of cytokines characteristics of the Th1 pattern of response.

All this phenotypical and molecular evidence points towards the induction of a CTL response subsequent to natural infection or immunisation with a proteoliposome from an extracellular bacteria, which is extremely surprising.

Other surprising experiences were obtained from the efficient incorporation of exogenous antigens such as Ovoalbumin (Ova) in the proteoliposome and its derivatives, although its abilities were not restricted to this antigen. The incorporation of Ova in the proteoliposome induced, in dendritic cells, the presentation of MHC class I level and its recognition and activation by T DC8+ cell hybridomes. These results point to the induction of a CTL response against heterologous antigens which may be used for the development of new vaccine preparations..

The insertion or conjugation of CTL epitops from Ova were incorporated into the proteioliposomes or its derivatives and thus increased the induced response against these epitops.

Proteoliposomes which are able to express high concentrations of the antigens of interest also form part of this invention, using genetic engineering of the productive proteoliposome strain to "more or less" express the amount of desired heterologous antigens.

"Antigen of interest" means those from fungi, virus, bacteria, protozoa and cancer cells that require CTL responses for their efficient elimination by the immune system, without any restriction to the antigens already identified.

"More or less" means specifically that the strain expresses more than 20, 15, 10, 5, 4, 3, 2, 1, 0.5 or 0.1 % of the quantity of the antigen or the antigens of interest. Preferably the modified strain by genetic engineering express from 0.5 to 10% of the antigen of interest.

The gene encoding for the antigen of interest of the invention can be modified by genetic engineering by previously mentioned

The obtention of different vaccine formulations exploiting Proteoliposome and cochlear structures abilities to induce CTL response, not previously described, also form part of this invention.

Associated tumorous, ganglioside or protein antigens from *Leishmania* were included in the proteoliposomes or its derivatives and the induction of CD8+ response and production of IFNy was demonstrated when Blab/c mice were immunised and challenged *in vitro* with the respective antigens. Also shown to be effective are vaccine formulations where the antigens are conjugated to a part of the protoeoliposomes of the vaccinal composition or once conjugated to these, are transformed into cochlear structures.

The vaccine formulations of the present invention can be used to protect a mammalian susceptible to an infection or to treat tumorous diseases by administering them systemically or mucosally. These applications may include intramuscular, intraperitoneal, intradermic, or subcutaneous injections or mucosal administrations by oral/feed or nasal/respiratory routes or genitourinary tract.

The amount of Proteoliposome or cochlear structures in each formulation is selected as the amount that shows adjuvant function, always being less than that used in the anti-meningoccocal vaccines based in these structures, with the result that the secondary effects are minor and not significant. This amount can vary depending on the antigen of interest and the way in which it is incorporated. Generally the amount of Proteoliposome or cochlear structures will be between 1 and 50 µg per dose and more typically between 5 and 25 µg. The amount of the antigen of interest will be in the range of 0.1 to 20% of the Proteoliposome or cochlear structure mass and preferably 0.5 to 10%.

The number of doses will chiefly depend on the type of vaccine formulation, whether it is prophylactic or therapeutic. In the first one, a maximum of three doses will be applied and in the second one, up to five doses can be applied. Both can be applied in children under one year of age, from 2 to 5 years, schoolchildren, adolescent, adults and elderly persons.

The innovation of this invention is the use of the Proteoliposomes or cochlear structures derived from outer membrane of Gram-negative bacteria and particularly from *N. meningitidis* B, as adjuvants inductors of CTL activity.

It is particularly novel that the natural infection with *N. meningitidis* B, extracellular bacteria, induced response of T CD8⁺ cells, lymphocytes more associated with cytotoxic responses, and that they produced IFNγ and IL-2, evidencing the induction of CTL activity. Thus, these lymphocytes are also increasing the Th1 response previously described.

Other novel aspect is its application in prophylactic formulations against fungal, viral, bacterial and protozoan infections and its therapeutic application in subjects affected by malignant tumours to induce cytotoxic response.

It is particularly novel, the conjugation or insertion of cytotoxic epitopes in the Proteoliposomes or cochlear structures increasing the CTL response.

The possibility of using these formulations by the mucosal route, besides the parenteral route and the combination of both is also novel.

The modification by genetic engineering of meningoccocal strains to produce Proteoliposomes or its derivatives expressing cytotoxic antigens is another novel aspect of this invention.

CTL response against proteoliposomes from extracellular bacteria and its derivatives, the cochlear structures, was unexpectedly obtained, when the induction of cytotoxic activity was only circumscribed to intracellular germs, tumours or certain adjuvants which permitted cross priming.

Another unexpected result was the finding of induction of CTL activity in those convalescing from meningococcic illnesses or carriers of *Neisseria,* an extracellular germ which should not induce CTL activity.

These activities were demonstrated by the induction of CD8+ lymphocytes, for the presence of IFNy and IL2 in them, the presentation of heterologous antigens in MHC class I of dendritic cells and their recognition and activation of CD8+ hybridomes and the cell lysis which express heterologous antigens.

The protoeoliposomes and cochlear structures are obtained on an industrial scale under Good Manufacture Practices conditions in the Finlay Institute and we possess methodology for the inclusion of heterologous antigens in the same. The proposed solution has the following advantages:
The immunologic effect achieved by these formulations allowed the induction of CTL response by the proteoliposome or the cochlear structures against the heterologous antigens incorporated into the same. This is applicable but not restricted to intracellular microorganism and tumor diseases.
The immunological effect of the proteoliposome as vaccine and transitively as adjuvant, where lower concentrations are used, is safe in children younger than 1 year, from 2 to 4 years, schoolchild, adolescents and adults.
The proteoliposomes and the cochlear structures also turn T-independent antigens (conjugated or covalently included) such as carbohydrates into T-dependent antigens due to the preferential cellular response (Th1) induced by them. This property is also applicable to antigens of interest of sacaridic nature.

The flexibility of the cochlear structures is useful for incorporating particulated antigens, especially AND inducing a CTL response against them.

The invention will be described through the following specific examples.

Example 1. Proteoliposome obtaining. For obtaining Proteoliposome a culture of *N. meningitidis* B or *Salmonella typhi* is performed and the biomass collected by centrifugation is subjected to a process of extraction with detergents, enzymes and ultrasound. Cellular debris is removed through centrifugation and the supernatant is subjected then to a digestion with nucleases to eliminate nucleic acids. The extract is recovered through ultracentrifugation, re-suspended in a solution with detergent and purified from the rest of components of low and medium molecular weight through a chromatography of molecular exclusion. The Pretroliposome obtained contain less than 10% of nucleic acids and less than 10% of inserted LPS in its structure but never free. The LPS is essential for giving off as danger signals to trigger the innate immune response. The final product is subjected to a group of biological and physico-chemical controls.

Example 2. T CD4⁺ and T CD8⁺ lymphocytes response in immunized mice. Balb/c mice were immunized with two doses of Proteoliposome or AFCo1, 25 µg each one, by intramuscular or nasal routes 14 days apart. Spleen cell were isolated 7 days after the second dose and incubated for 4 days with the Proteoliposome. The expanded cells were treated with anti-CD4 or anti-CD8 monoclonal antibodies and subsequently with a florescent anti-IgG and were evaluated by Flow Cytometry. The immunization stimulated both T CD4⁺ and T CD8⁺ lymphocytes anti-Proteoliposome. 45% of lymphocytes were T CD4⁺ and 40% were T CD8⁺.

Example 3. T CD4⁺ and T CD18⁺ lymphocytes response in immunized humans.

Young adults were immunized with two doses of VA-MENGOC-BC™, 50 µg each one, 6 weeks spaced, by intravenous administration. The periferic mononuclear cells were purified over Ficoll-Hipaque from blood sample taken 7 days after the second dose. For 4 days these cells were incubated with proteoliposome. The expanded cells were treated with monoclonal antibodies anti-CD4 or anti-CD8 and subsequently labelled with an anti-IgG to be evaluated by Flow Cytometry. The immunization stimulated both T CD4⁺ and T CD8⁺ lymphocytes anti-Proteoliposome. 50% of lymphocytes were T CD4⁺ whiles 42% were T CD8⁺.

Example 4. T CD4⁺ and T CD8⁺ lymphocytes response in convalescents from meningoccocal disease. PBMC were purified over Ficoll-Hipaque from blood sample taken from individuals convalescents from meningoccocal disease caused by *N. meningitidis* B two months after they had been discharged. These were incubated 4 days with the Proteoliposome. The expanded cells were treated with anti-CD4 or anti-CD8 monoclonal antibodies and subsequently with a florescent anti-IgG and evaluated by Flow Cytometry. A proliferation of T CD4⁺ and T CD8⁺ lymphocytes occurred, 47% of the lymphocytes were T CD4⁺ and 39% were T CD8⁺.

Example 5. Activation signals and intracellular cytokines production by lymphocytes from immunised humans. Young adults were immunised with two doses of VA-MENGOCBC® each of 50 µg by intramuscular administration, in a space of 6 weeks. Several months after the second dose a blood sample was taken. The samples were incubated for 27 hours with proteoliposome at a concentration of 10 or 20 µg/ml. Later, red cells were lysed and the other cells were treated to become permeable to monoclonal antibodies for detection of intracellular cytokines (IFN-γ, IL2 or IL5) and surface markers (CD4, CD8 or CD69). 3.28% of T CD4⁺ and 20.65% T CD8⁺ lymphocytes were activated with Proteoliposome 10 µg/ml while 3.86% T CD4⁺ and 14.11% T CD8⁺ lymphocytes were activated with Proteoliposome at 20 µg/ml. Nevertheless, only the activated T CD8⁺ lymphocytes produced IFN-γ and IL-2, although a moderate production of IL-2 was also detected in activated T CD4. The percentages of intracellular IFN*y* in the T CD4⁺ and T CD8⁺ lymphocytes with 10 µg/ml were 0.35 and 0.89 and with 20 µg/ml 1.54 and 1.29 respectively, but of the IFNy productive cells only 0.57% were activated with 10 µg/ml and 0.87% with 20 µg/ml of the T CD8+. The percentages of lymphocytes producing intracellular IL-2 were 1.19% T CD4⁺ and 1.07% T CD8⁺ at 10 µg/ml respectively and 1.18 % T CD4⁺ and 1.6% T CD8⁺ at 20 µg/ml .However, only the IL2 producing cells were activated at 0.68 and 0.7% with 10 and 20µg/ml, respectively of the T CD8+ lymphocytes. In the case of the T CD8+ lymphocytes only low IL2 production percentages in activated cells were detected. No IL5 production was observed by any of the lymphocyte subpopulations..

Example 6. Activation signals and intracellular cytokine production by lymphocytes from patients convalescent from meningoccocal disease. Blood samples were taken from individuals convalescent from meningoccocal disease caused by *N. meningitidis* B two months after the patients were discharged. These were incubated with Proteoliposome at 10 µg/ml for 27 hours. Later, red cells were lysed and the other cells were treated to become permeable to monoclonal antibodies for detection of intracellular cytokines (IFN-γ, IL2 or IL5) and surface markers (CD4, CD8 or CD69). 9.29% T CD4⁺ and 26.28% T CD8⁺ lymphocytes were activated. Both subsets of activated lymphocytes produced IFN-γ and just T CD8⁺ produced IL-2. 3.46% T CD4⁺ and 1.36% T CD8⁺ lymphocytes produced intracellular IFN-γ but of these just 0.65% and 0.95% T respectively were activated. The percentages of lymphocytes producing intracellular IL-2 were 0.73% T CD4⁺ and 0.93% T CD8⁺ but of these just 0.73% and 0.73% respectively, were activated. Production of IL-5 was not detected.

Example 7. Inclusion of exogenous antigens in the proteoliposome. Ovalbumin (Ova) was included in the proteoliposome through the use of detergents, particularly deoxycholate, which allowed the disruption of the proteoliposomic structure. Detergent elimination and the re-assembly of the structure in the presence of Ova allowed for its incorporation into the proteoliposome. The final proportion of proteoliposome: Ova was 100:11.2. The vesicular structure was verified using molecular exclusion chromatography resulting in similar profiles of proteoliposome alone and that which contained the Ova. The presence of Ova was confirmed using SDS-PAGE followed by Western blotting revealed with a polyclonal anti-ova antibody which demonstrated the presence of a band corresponding to ova within the proteoliposome proteic profile.

Example 8. Inclusion of exogenous antigens in cochlear structures. The inclusion of proteins from promastigotes and amastigotes of *Leishmania* was efficiently performed using the inclusion of the same in the solution during the process of formation of cochlear structures .The immunization in animals of cochlear structures. containing these antigens evidenced an increase of the anti-*Leishmania* immune response and the reduction of the indurations produced by the infection with this protozoa. The presence of this protein was also evidenced by SDS-PAGE followed by Western Blot revealed with anti *Leishmania* antibodies.

Example 9. Conjugation of antigens to the proteoliposome. This is described in the Example 1.2 of the patente "Método de obtención de vacunas conjugadas. Composiciones vacunales" Patente OCPI 2002-0257 de 14/11/02.

Example 10. Inclusion of PAMPs. LPS from *Neisseria meningitidis* B have been included in different quantities, increasing the normal concentration of it in the proteoliposome up to 12%. It has increased the immune response obtained against the proteoliposome. LPS from *Vibrio cholerae* have been also included in the proteoliposome inducing increased immune response against this antigen.

Example 11 IFN-γ Production by T CD8⁺ lymphocytes against heterologous antigens included in or conjugated with proteoliposomes and cochlear structures. Spleen cells from animals immunized with proteoliposomes including Ova or *Leishmania* proteins in cochlear structures induced T CD8+ response and these expressed IFNy on evaluation by the ELISPOT T assay. The conjugates of polysacharyde C to the proteoliposome induced IFN-γ determined by ELISÃ

Example 12. Inclusion of plasmid DNA in the cochlear structures and liberation of the same in the cellular cytoplasm. The plasmid pGFP, encoding the green fluorescent protein under an expression promoter from superior cells (CMVp), was efficiently included in cochlear structures. The cochlear structures containing this plasmid was added to a culture of L929 cell line. The expression of the green fluorescent protein in the cellular cytoplasm was evidenced through an optic fluorescence microscope.

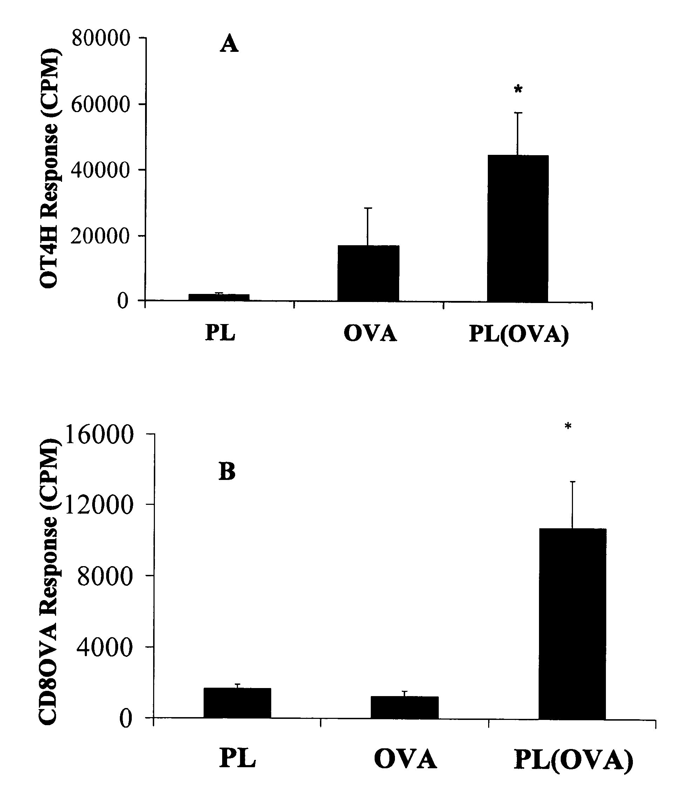

Example 13. Dendritic cells exposed to Proteoliposome (PL) containing Ovalbumin (Ova) can present Ova peptides to T-cells. MHC class II restricted CD4⁺ or MHC class I restricted CD8⁺ T-hybridoma cells that are specific for Ova (257-264) and Ova (265-277) peptides, respectively were used. Dendritic cells (DC) were incubated for 2 h with PL-OVA or soluble OVA and then co-cultured with T-cell hybridomas. Supernatants were collected after 24 h and tested for IL2 production using [³H]thymidine incorporation by the IL2-dependent CTLL cell line. IL2 production was used as a measure of the activation of the Ova-specific cell hybridomas. Figure 1 shows that DC pulsed with PL-Ova can present Ova antigen to both CD4⁺ and CD8⁺ T-cell hybridomas. T-cell hybridomas produced significantly higher levels of IL2 when stimulated with DC that had been pulsed with PL-Ova as compared to DC pulsed with Ova alone. Significant differences between the levels of presentation of specific Ova peptides in MHC-I and MHC-II by DC incubated with PL(Ova) or Ova were determined by Duncan's multiple comparison test with 95% confidence level. Thus, Proteoliposome can deliver antigens to DC for efficient antigen presentation to T-cells.

### Brief description of the figures

Figure 1. DC can process Ova peptides from Ova included in proteoliposome (PL-Ova) for MHC-I and MHC-II presentations. Fig. 1A shows the OT4H T-hybridoma cells response to DC from C57BL/6 mice co-incubated with PL, Ova or PL-Ova; Fig. 1 B shows the OvaCD8⁺ T-hybridoma cells response. IL-2 production from the hybridomas was quantified by [³H] thymydine incorporation by the IL-2-dependent CTLL cell line. Data are presented as the mean ± SD of three different experiments.
   *significantly different from other groups.

## Claims

1. Vaccine adjuvants **characterized by** a proteoliposomic structure or derivatives thereof, capable of inducing CTL response.

2. Vaccine adjuvants like in claim 1, **characterized** because the proteoliposome of its derivatives are of bacterial origin.

3. Vaccine adjuvants like in claim 2, **characterised** because they come from *Neisseria* or *Salmonella* genus

4. Vaccine adjuvants like in claim 1, **characterised** because the proteoliposome or its derivatives express heterologous antigens of interest from a strain modified by genetic engineering.

5. Vaccine formulation **characterized by** including the adjuvants described in claims 1 to 4, one or more antigens of interest as well as suitable excipients.

6. Vaccine formulation like in claim 5, **characterized by** the insertion of the antigen (s) of interest in the lipidic bilayer of the proteoliposomes being also present in its derivatives.

7. Vaccine formulation like in claim 5, **characterized by** the conjugation of the antigen (s) of interest to the proteoliposomes being also present in its derivatives.

8. Vaccine formulation like in claim 5, **characterized by** the co-administration of the antigen (s) of interest with the proteoliposomes or its derivatives.

9. Vaccine formulation like in claim 5, **characterized by** the antigen (s) can be selected from the group of antigens coming from fungi, virus, bacteria, protozoa and cancer and which have demonstrated the need for CTL response for their effective elimination by the immune system..

10. Vaccine formulation like in claim 5, **characterized by** a concentration rank of the proteoliposomes or its derivatives between 1 and 50 µg, particularly between 5 and 25 µg.

11. Vaccine formulation like in claim 5, **characterized by** a concentration rank of the antigen (s) of interest from 0.1 to 20% of the mass of the proteoliposomes or its derivatives, particularly from 0.5 to 10%.

12. Vaccine formulation like in claims 5 to 10, **characterised by** the administration route which may be selected from intramuscular, intraperitoneal, intradermical, subcutaneous or mucosal by oral/feed or nasal/respiratory routes or by genitourinary tract.

13. The use of vaccine formulation like in claims 5 to 11 to protect mammalians susceptible to infections and to treat tumorous diseases.

14. Immunization schedule using vaccine formulation like in claims 5 to 11, **characterized by** the application of three doses as maximum to achieve a prophylactic effect and five doses as maximum to achieve a therapeutic effect.
